# EUROPÄISCHE PATENTSCHRIFT

(11) **EP 3 258 982 B1**
(45) Veröffentlichungstag und Bekanntmachung des Hinweises auf die Patenterteilung: **10.08.2022**
(21) Anmeldenummer: 16703550.0
(22) Anmeldetag: 09.02.2016
(51) Int. Cl.: A61M 1/06

(54) **BRUSTHAUBE MIT FLEXIBLEM RAND**
BREAST CUP WITH FLEXIBLE EDGE
TIRE-LAIT DOTÉ D'UN BORD SOUPLE

(30) Priorität: 20.02.2015 EP 15155897
(43) Veröffentlichungstag der Anmeldung: 27.12.2017
(60) Teilanmeldung: 22182576.3
(73) Patentinhaber: Medela Holding AG, 6340 Baar (CH)
(72) Erfinder: FURRER, Etienne, 6330 Cham (CH); SCHLIENGER, André, 8933 Maschwanden (CH); MUTHER, Marcel, 6030 Ebikon (CH)
(74) Vertreter: Clerc, Natalia
(86) Internationale Anmeldenummer: PCT/EP2016/052716
(87) Internationale Veröffentlichungsnummer: WO 2016/131678

(56) Entgegenhaltungen:
- GB-A- 2 138 686
- US-A1- 2006 106 334
- US-A1- 2009 254 029
- US-B1- 6 383 164
- US-B2- 8 444 596

## Beschreibung

### TECHNISCHES GEBIET

Die vorliegende Erfindung betrifft eine Brusthaube mit einem flexiblen Rand für eine Brustpumpe zum Abpumpen von menschlicher Muttermilch.

### STAND DER TECHNIK

Brustpumpensysteme zum Abpumpen von menschlicher Muttermilch sind wohlbekannt. Sie weisen eine manuell oder elektromotorisch betriebene Vakuumpumpe, mindestens eine Brusthaube zur Auflage auf die Mutterbrust, einen Adapter und einen Milchsammelbehälter auf, in welchem die abgepumpte Milch aufgefangen wird. Die Brusthaube ist mit der Vakuumpumpe direkt oder über eine Saugleitung verbunden, so dass in der Brusthaube ein zyklisch variierender Unterdruck an die Brust angelegt werden kann, um die Milch aus der Brust abzupumpen. Der Adapter hält die Brusthaube und verbindet sie mit der Vakuumpumpe bzw. der Saugleitung. Der Adapter ermöglicht zudem die Verbindung mit dem Milchsammelbehälter, entweder durch eine direkte Kopplung des Behälters an den Adapter oder über eine Milchleitung.

Bekannte Brusthauben weisen einen Trichter und ein Anschlussteil zur Verbindung mit dem Adapter auf. Üblicherweise sind die Trichter kegelstumpfförmig ausgebildet, wobei sie steif oder weich sein können. Steife Trichter können zudem mit einer weichen Einlage versehen sein.

Der Trichter sollte möglichst dicht an der Mutterbrust anliegen, damit eine wirkungsvolle Pumpkammer durch Mutterbrust und Brusthaube gebildet werden kann. Zudem sollte er angenehm auf der Mutterbrust aufliegen, um ein möglichst druckstellenfreies und entspanntes Abpumpen zu ermöglichen. Die Grösse und Form der menschlichen Mutterbrust ist zudem sehr stark vom Individuum abhängig und entsprechend unterschiedlich. Aus dem Stand der Technik sind verschiedene Brusthauben bekannt, welche mindestens einem Teil dieser Probleme Rechnung tragen wollen.

US 6 387 072 und US 6 723 066 offenbaren zum Beispiel ein Brusthaubenset mit einer steifen Haubenbasis und einem Set von verschieden grossen, in die Basis einsetzbaren Brusthauben. Die Trichter der Brusthauben sind kegelstumpfförmig ausgebildet, wobei in einer Ausführungsform der umlaufende Rand eine Ausnehmung aufweist. Diese Brusthaube kann so auf die Brust gelegt werden, dass die Ausnehmung über einen empfindlichen oder anatomisch speziell geformten Bereich der Mutterbrust zu liegen kommt und dieser Bereich somit geschont wird.

US 2006/0116632 schlägt vor, das Anschlussteil der Brusthaube in einem Winkel zu einem kegelförmigen Trichter anzuordnen.

US 6 673 037 zeigt eine steife Brusthaube mit einem Trichter, welcher einen elliptischen Grundriss aufweist. Auf der Innenseite des Trichters sind Erhebungen vorhanden, welche auf die Brust einwirken.

WO 2011/035488 offenbart eine relativ kleine, sehr flexible Brusthaube, deren Öffnungswinkel sich der Brust anpasst.

US 4 772 262 offenbart eine weiche Brusthaube mit einer elliptischen Öffnung.

US 7 413 557 beschreibt eine Brusthaube mit einem steifen Trichter und einer darin angespritzten weichen Einlage. Die Einlage ist über den Rand des Trichters hinaus als weicher asymmetrischer Kragen weitergezogen. Der Kragen weist eine Oberseite zur Anlage auf der oberen Seite der Brust und eine Unterseite zur Anlage auf der unteren Seite der Brust auf. Diese Brusthaube weist den Nachteil auf, dass sie für kleine Brüste zu gross ist und bei speziell geformten Brüsten ebenfalls nicht optimal anliegt. Des Weiteren ist ihre Herstellung kostenintensiv, da eine relativ grosse Menge Silikon verwendet werden muss.

GB 2 138 686 offenbart einen zweigeteilten Trichter mit einem weichen, asymmetrischen brustnahen Teil. Dieser Teil ist auswechselbar, so dass er der Grösse der Mutterbrust entsprechend ausgewählt werden kann. Da er jedoch auf die Trichterbasis aufgesteckt werden muss, wirkt sich der Verbindungsbereich störend auf den Komfort der Mutter aus.

US 8 444 596 schlägt vor, in einem Zweikomponenten-Spritzgussverfahren eine weiche Lippe an den steifen Trichter anzuspritzen.

GB 2 138 686 und US 2006/0106334 offenbaren Brusthauben mit einem aufgesteckten Kragen. US 2009/0254029 zeigt eine Brusthaube mit einem Liner. US 6 383 164 beschreibt eine Brusthaube mit einem Trichter, dessen Innenseite beschichtet ist.

Obwohl einige der bekannten Lösungen bereits gute Akzeptanz bei Müttern finden, weisen sie nach wie vor Nachteile bezüglich Anpassungsfähigkeit an die individuelle Grösse und Form der Mutterbrust und bezüglich dichtendes Anliegen an die Mutterbrust auf. Ein Problem liegt darin, dass die Mutterbrust nicht nur individuell verschieden ist, sondern sich während des Abpumpens selber ebenfalls verändert. Eine gefüllte Brust ist hart und gross, während des Abpumpens wird sie weicher und kleiner. Die Druckempfindlichkeit des Brustgewebes und somit die Reaktion der Mutter auf die angelegte Brusthaube verändert sich entsprechend. Weiche Brusthauben sind in der Fertigung teurer als steife Brusthauben. Kombinierte Brusthauben mit einem steifen Trichter und einem weichen Rand sind in den bisher vorgeschlagenen Lösungen jedoch relativ aufwändig in der Herstellung und entsprechend kostenintensiv.

### DARSTELLUNG DER ERFINDUNG

Es ist deshalb eine Aufgabe der Erfindung, eine Brusthaube zu schaffen, welche sich der Mutterbrust während des Abpumpens anpasst und möglichst kostengünstig gefertigt werden kann.

Diese Aufgabe löst eine Brusthaube mit den Merkmalen des Anspruchs 1.

Die erfindungsgemässe Brusthaube für eine Brustpumpe zum Abpumpen von menschlicher Muttermilch weist ein steifes Basisteil mit einem rohrförmigen Stutzen und mit einem einstückig daran angeformten, sich vom Stutzen weg erweiternden Trichter zur Aufnahme einer Mutterbrust auf. Die Brusthaube umfasst ferner einen flexiblen Kragen, welcher an einem dem Stutzen abgewandten Ende des Trichters am Trichter angeordnet ist und welcher zur Anlage auf die Mutterbrust ausgebildet ist. Das genannte Ende des Trichters bildet eine erste Anlagefläche. Der Kragen weist ein dem Trichter zugewandtes Ende auf, welches eine zweite Anlagefläche bildet, wobei die erste und die zweite Anlagefläche in einer umlaufenden Verbindungsnaht stumpf aneinander grenzen. Der Trichter weist im Bereich der Verbindungsnaht eine erste Wandstärke und der Kragen eine zweite Wandstärke auf, welche annähernd gleich gross ist wie die erste Wandstärke. Vorzugsweise sind die erste Wandstärke und die zweite Wandstärke genau gleich gross, d.h. sie sind genau gleich dick.

Diese Brusthaube lässt sich einfach herstellen. Die zwei Anlageflächen sind über die Verbindungsnaht nicht zerstörungsfrei lösbar miteinander verbunden und bilden eine stumpfe Stossverbindung. Die Verbindung zwischen den zwei Anlageflächen kann auf einfachste Art und Weise stoffschlüssig erfolgen. Insbesondere kann die Brusthaube in einem Zweikomponenten-Spritzgussverfahren hergestellt werden.

In bevorzugten Ausführungsformen weist der Trichter einen ersten Öffnungswinkel auf und der Kragen im unbelasteten Zustand einen zweiten Öffnungswinkel, wobei der zweite Öffnungswinkel kleiner ist als der erste Öffnungswinkel. Der Kragen ist im unbelasteten Zustand vorzugsweise in Bezug auf den Trichter nach innen geneigt. Der Kragen lässt sich vorzugsweise beim Anlegen an die Mutterbrust nach aussen drücken und vergrössert somit elastisch seine Aufnahmeöffnung. Diese Merkmale führen einzeln und in Kombination miteinander dazu, dass bereits ein minimaler Anpressdruck genügt, damit der Kragen der Brusthaube dichtend auf der Brust anliegt. Die Brusthaube kann auch an empfindliche Brüste schonend und sanft angelegt werden. Zudem passt sie sich dank dieser Merkmale auch während des Abpumpens an die sich verändernde Brust an, ohne dass die Mutter den Anpressdruck allzu stark verändern muss. Des Weiteren ist die Brusthaube auch für kleine Brüste eher geeignet.

In einer bevorzugten Ausführungsform weist der Kragen mindestens, vorzugsweise genau zwei Symmetrieebenen auf. Dies erhöht die Passform der Brusthaube auf der Brust und sie lässt sich optimal dichtend auf die Brust auflegen. Zudem lässt sie sich in eine Position bringen, welche der individuellen Form und Grösse der Mutterbrust angepasst ist. Dies gilt sowohl bei aufrechter wie auch zurück gelehnter Körperhaltung der Mutter.

In bevorzugten Ausführungsformen weist der Kragen ein dem Trichter abgewandtes Ende auf, welches einen Auflagerand zur Auflage an die Mutterbrust bildet, wobei der Auflagerand eine Raumkurve ist, welche nicht in einer Ebene verläuft. Diese Brusthaube liegt dank ihrer speziellen räumlichen Kurvenform optimal an der Brust auf. Die spezielle Form ermöglicht es, die Brusthaube in verschiedenen Drehpositionen an die Brust zu legen. Die Mutter kann die für sie und ihre Brustform optimale Drehposition wählen. "Optimal" ist in diesem Text im Sinne von bestmöglich dicht und bestmöglich druckentlastend zu verstehen. Da die Mutter die Brusthaube in eine andere Position drehen kann, ist es ihr zudem möglich, Druckstellen zu entlasten. Des Weiteren wirkt die Brusthaube mit der räumlichen Kurvenform ergonomischer und femininer. Allfällige psychologische Hürden der Mutter werden dadurch abgebaut, was wiederum den Erfolg beim Abpumpen erhöht.

Vorzugsweise weist nicht nur der Trichter, sondern auch oder alternativ die Auflagekante mindestens zwei oder genau zwei Symmetrieebenen auf. Der Auflagerand weist in einer optimalen Ausführungsform in einer Ebene senkrecht zu einer Längsachse der Brusthaube eine Projektion auf, welche die Form einer Ellipse hat. In alternativen Ausführungsformen ist diese Projektion ein geschlossener Polygonzug mit abgerundeten Ecken und nach innen geneigten Seiten. Der Gesamteindruck ist blumenförmig.

Vorzugsweise ist der Kragen ein Kegelstumpf und noch bevorzugter ein gerader Kreiskegelstumpf. Dasselbe gilt für den Trichter. Die Kombination dieser Form mit einer nichtebenen Raumkurve als Auflagerand führt zu einer Brusthaube mit einer optimalen Passform. Infolgedessen ergibt sich ein stetig zunehmendes und zonenweise elastisches Anschmiegen der Brusthaube an die Mutterbrust.

In bevorzugten Ausführungsformen weist der Kragen in seinem unbelasteten Zustand in Richtung der Längsmittelachse der Brusthaube einen gleichbleibenden Öffnungswinkel auf, mindestens bis in einen Bereich eines dem Trichter abgewandten Endes des Kragens. Das heisst, der Auflagerand kann durchaus einen anderen Winkel aufweisen und beispielsweise stärker nach aussen geneigt sein, um eine vergrösserte Auflagefläche zu bilden.

Erfindungsgemäss ist die erste Wandstärke im Bereich der Verbindungsnaht im Vergleich zu einer angrenzenden Wandstärke des Trichters vergrössert und/oder die zweite Wandstärke des Kragens ist im Bereich der Verbindungsnaht im Vergleich zu einer angrenzenden Wandstärke des Kragens vergrössert. Diese Verdickung der Wandstärken im Bereich der Verbindungsnaht führt dazu, dass die Brusthaube trotzdem robust ist und der Kragen genügend abreiss-sicher am Trichter befestigt ist. Zudem bildet diese Verdickung ein haptisches Element zur Verbesserung der Handhabung. Die Brusthaube lässt sich dank der Verdickung mit relativ wenig Materialverbrauch fertigen. Dies führt zu einer leichten und für die Mutter deshalb angenehm anzuwendenden Brusthaube. Zudem reduziert dies die Herstellungskosten.

Vorzugsweise sind beide Wandstärken im Bereich der Verbindungsnaht im Vergleich zu ihren angrenzenden Wandstärken vergrössert bzw. sie sind dicker ausgebildet.

Die Wandstärke des Trichters im Bereich der ersten Wandstärke und/oder die Wandstärke des Kragens im Bereich der zweiten Wandstärke vergrössern sich stufenlos oder in einer Stufe. Je nach Ausführungsform können beide stufenlose Vergrösserungen aufweisen oder sie können sich beide in einer Stufe vergrössern. In einer anderen Ausführungsform erfolgt eine Vergrösserung stufenlos und die andere erfolgt in einer Stufe.

Vorzugsweise verläuft die Verbindungsnaht nicht in einer Ebene. Vorzugsweise weist das brustseitige Ende des Trichters ebenfalls eine nichtebene Raumkurve auf. Vorzugsweise weist die Verbindungsnaht eine stetige Form auf. In anderen Ausführungsformen verläuft sie jedoch unstetig. Die Verbindungsnaht kann auch in einer Ebene verlaufen.

Vorzugsweise besteht die Brusthaube lediglich aus diesen zwei Bereichen: d.h. aus dem einstückigen Basisteil und dem einstückigen Kragen.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung einer erfindungsgemässen Brusthaube in einer ersten Ausführungsform;
- Figur 2: einen Längsschnitt durch die Brusthaube gemäss Figur 1;
- Figur 3: eine brustnahe Ansicht der Brusthaube gemäss Figur 1 mit einer Form einer Verbindungsnaht in einer ersten Ausführungsform;
- Figur 4: einen Längsschnitt durch eine erfindungsgemässe Brusthaube in einer zweiten Ausführungsform;
- Figur 5: einen Längsschnitt durch eine erfindungsgemässe Brusthaube in einer dritten Ausführungsform;
- Figur 6: eine brustnahe Ansicht der Brusthaube gemäss Figur 5;
- Figur 7: einen anderen Längsschnitt durch die Brusthaube gemäss Figur 5;
- Figur 8a: einen Schnitt durch den Bereich einer Verbindungsnaht mit Wandstärken des Trichters und des Kragens in einer ersten Ausführungsform;
- Figur 8b: eine Variante zu Figur 8a;
- Figur 9a: einen Schnitt durch den Bereich einer Verbindungsnaht mit Wandstärken des Trichters und des Kragens in einer zweiten Ausführungsform;
- Figur 9b: eine Variante zu Figur 9a;
- Figur 10a: einen Schnitt durch den Bereich einer Verbindungsnaht mit Wandstärken des Trichters und des Kragens in einer nicht beanspruchten Ausführungsform;
- Figur 10b: eine Variante zu Figur 10a;
- Figur 11: eine Ansicht auf eine Brusthaube mit einer Form einer Verbindungnaht in einer zweiten Ausführungsform;
- Figur 12: eine Ansicht auf eine Brusthaube mit einer Form einer Verbindungnaht in einer dritten Ausführungsform;
- Figur 13: einen Längsschnitt durch eine erfindungsgemässe Brusthaube vor dem Anlegen auf eine Mutterbrust mit Blick von der Seite;
- Figur 14: den Längsschnitt durch die Brusthaube gemäss Figur 13 nach dem Anlegen auf eine Mutterbrust;
- Figur 15: einen Längsschnitt durch die Brusthabe gemäss Figur 13 vor dem Anlegen an die Mutterbrust mit Blick von oben;
- Figur 16: eine Seitenansicht einer erfindungsgemässen Brusthaube in einer weiteren Ausführungsform;
- Figur 17: die Brusthaube gemäss Figur 16 in einer brustnahen Ansicht;
- Figur 18: eine perspektivische Darstellung der Brusthaube gemäss Figur 16;
- Figur 19: eine erste Seitenansicht einer erfindungsgemässen Brusthaube in einer weiteren Ausführungsform;
- Figur 20: eine zweite Seitenansicht der Brusthaube gemäss Figur 19;
- Figur 21: die Brusthaube gemäss Figur 19 in einer brustfernen Ansicht und
- Figur 22: eine perspektivische Darstellung der Brusthaube gemäss Figur 19.

### BESCHREIBUNG BEVORZUGTER AUSFÜHRUNGSFORMEN

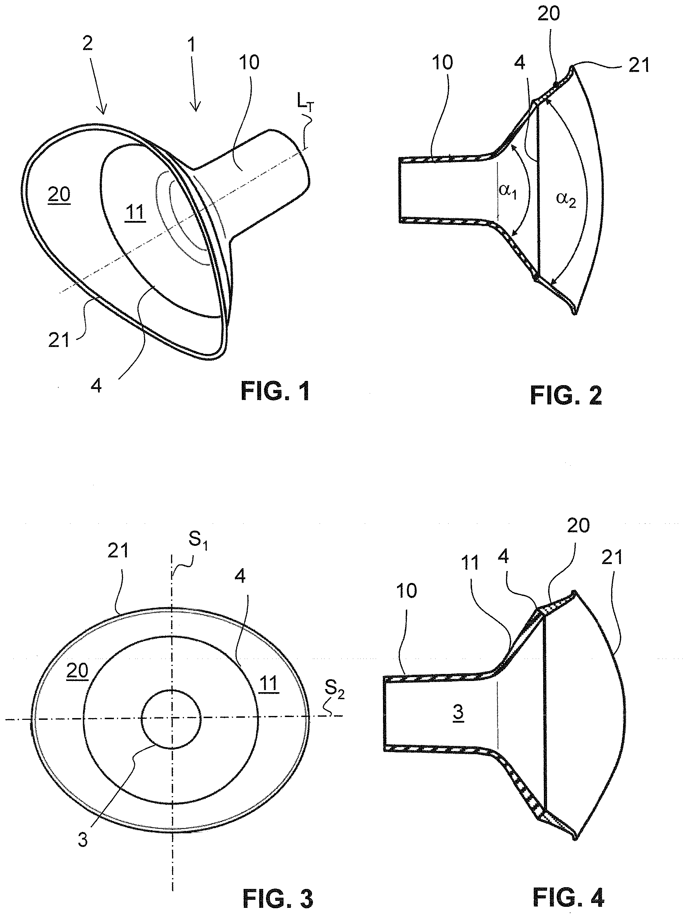

In den Figuren 1 bis 3 ist ein erstes Ausführungsbeispiel einer erfindungsgemässen Brusthaube dargestellt. Sie weist ein starres oder steifes Basisteil 1 und einen daran angeformten flexiblen und vorzugsweise weichen Kragen 2 auf. Das Basisteil 1 und der Kragen 2 bilden gemeinsam einen Kanal oder einen Durchgang 3, damit ein von einer Vakuumpumpe zyklisch erzeugter Unterdruck an die Mutterbrust angelegt und abgepumpte Milch von der Mutterbrust in einen Milchsammelbehälter fliessen kann.

Das Basisteil 1 und der Kragen 2 sind vorzugsweise aus Kunststoff gefertigt, wobei sie vorzugsweise aus unterschiedlichen Materialien bestehen. Das Basisteil 1 ist vorzugsweise aus Polypropylen (PP) gefertigt, der Kragen 2 aus einem thermoplastischen Elastomer (TPE). Sie sind vorzugsweise gemeinsam einstückig in einem Zweikomponenten-Spritzgussverfahren hergestellt.

Das Basisteil 1 weist einen rohrförmigen Stutzen 10 zur Verbindung mit einem hier nicht dargestellten Adapterteil eines Brustpumpensystems auf. Derartige Adapter oder Kopplungsteile sind hinlänglich bekannt. Sie halten die Brusthaube und verbinden die Brusthaube mit der Vakuumpumpe und dem Milchsammelbehälter.

Der Stutzen 10 geht einstückig in einen Trichter 11 über, welcher sich vom Stutzen 10 zur Brust hin erweitert. Der Trichter 11 ist in den hier beschriebenen Beispielen ein gerader Kreiskegelstumpf. Er weist vorzugsweise einen ersten Öffnungswinkel α₁ von 90° bis zu 120° und vorzugsweise von 105° auf.

Der steife Trichter 11 weist vorzugsweise auf seiner Innenseite und/oder auch auf seiner Aussenseite keinerlei Erhebungen oder Vertiefungen auf. Seine innere Fläche und seine äussere Fläche sind somit vorzugsweise glatt ausgebildet. Dies führt zu einer optimalen Gleitpaarung mit der Haut. Die glatte äussere Fläche kann jedoch rau ausgebildet sein, um genügend Griffigkeit für die Mutter zu erhalten. Sie kann auch mit haptischen Elementen versehen sein, welche der Mutter die Drehpositionierung der Brusthaube erleichtert.

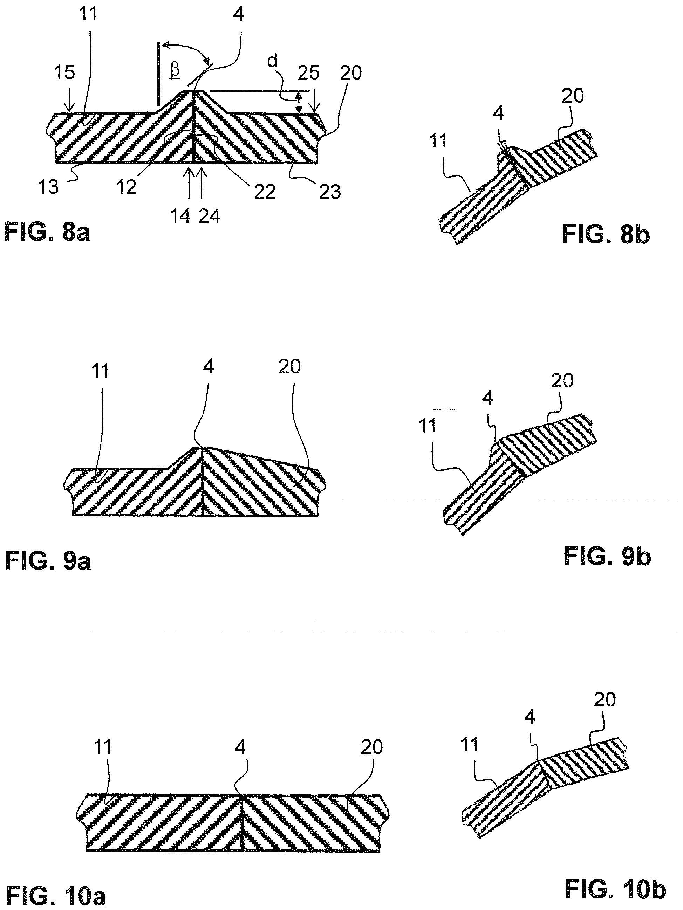

Der Trichter 11 weist ein dem Stutzen 10 fernes Ende auf, welches eine erste Anlagefläche 12 bildet. Diese erste Anlagefläche 12 ist in Figur 8 erkennbar.

Der weiche Kragen 2 weist einen Grundkörper 20 auf mit einem brustfemen Ende, welches über eine Verbindungsnaht 4 mit dem Trichter 11 verbunden ist und einem brustnahen Ende, welches einen Auflagerand 21 zur Auflage auf die Mutterbrust bildet.

Der Grundkörper 20 weist in diesem Beispiel die Form eines geraden Kreiskegelstumpfes auf. Er endet brustnah in einer in sich geschlossenen Raumkurve, d.h. in einer Kurve, welche nicht in einer Ebene liegt. Diese Raumkurve bildet den Auflagerand 21.

Der Auflagerand 21 ist vorzugsweise flächig ausgebildet, damit er nicht nur linienförmig sondern flächig an der Brust anliegt. Dies lässt sich beispielsweise durch Verdickung des Auflagerandes 21 im Vergleich zur restlichen Wandung des Kragens 2 erreichen oder der Auflagerand 21 kann, wie in Figur 2 dargestellt, nach aussen gebogen ausgebildet sein.

Die Raumkurve des Auflagerandes 21 kann beliebig geformt sein. In diesem Beispiel ist ihre Projektion senkrecht zur Längsmittelachse L_{T} der Brusthaube eine Ellipse, wie dies in Figur 3 erkennbar ist. Die Projektion weist somit genau zwei Symmetrieachsen S₁ und S₂ auf. Der Auflagerand 21 und somit der Kragen 2 weisen genau zwei Symmetrieebenen auf, welche die zwei Symmetrieachsen beinhalten und welche senkrecht zur Zeichnungsblattebene verlaufen. Diese Symmetrieebenen sind deshalb ebenfalls mit dem Bezugszeichen S₁ und S₂ versehen.

Der Kragen 2 kann eine beliebige Form aufweisen. Sein Auflagerand 21 kann eine Raumkurve oder eine ebene Kurve sein. Sein Grundkörper 20 kann beispielsweise die Form eines geraden Kegels mit einer elliptischen Grundform oder eines schiefen Kegels aufweisen. Er kann auch eine andere Form besitzen, z.B. eine asymmetrische Form. Vorzugsweise weist er jedoch mindestens zwei Symmetrieachsen bzw. -ebenen auf. Die hier dargestellten Raumkurven mit auf die Achse L_{T} zentrierten elliptischen Projektionen sind jedoch bevorzugt.

Der kreiskegelstumpfförmige Grundkörper 20 weist einen Öffnungswinkel α₂ auf, welcher vorzugsweise kleiner ist als der Öffnungswinkel α₁ des Trichters 11. Der zweite Öffnungswinkel α₂ ist vorzugsweise zwischen10° bis 40° kleiner als α₁ bei einem Winkel α₁ von 105°. Vorzugsweise beträgt α₂ annähernd 80°.

Der Kragen 2 ist im unbelasteten Zustand vorzugsweise gegenüber dem Trichter 11 nach innen geneigt. Ist der Grundkörper 20 ein Kegelstumpf mit einem elliptischen Grundriss, so ist sein zweiter Öffnungswinkel α₂ über mindestens einen Teil seines Umfangs, vorzugsweise über den gesamten Umfang kleiner als der Öffnungswinkel α₁ des Trichters 11. Die Öffnungswinkel α₁, α₂ sowohl des Trichters 11 wie auch des Kragens 2 bleiben vorzugsweise in Richtung der Längsmittelachse L_{T} der Brusthaube und somit entlang der Falllinien der inneren Oberflächen des Trichters 11 bzw. des Kragens 2 unverändert.

Das dem Trichter 11 zugewandte erste Ende des Kragens 2 weist dieselbe Form auf wie das benachbarte Ende des Trichters 11. Hier ist es somit ebenfalls kreisförmig. Es bildet eine zweite Anlagefläche 22, welche in Figur 8 erkennbar ist.

Die Figur 8a stimmt nicht mit den Abbildungen gemäss Figur 2 überein. In Figur 2 ist der Kragen 2 nach innen geneigt und die Verbindung bzw. der Stoss gebildet durch Kragen 2 und Trichter 11 erfolgt in einem Winkel. Dieser Winkel beträgt vorzugsweise 10° bis 30°. In den Figuren 8a, 9a und 10a hingegen ist die Verbindung in einer Ebene und die zwei inneren Oberflächen 13, 23 des Trichters 11 und des Kragens 2 fluchten miteinander. Eine geradlinige Verbindung ist möglich, wobei die winklige Verbindung bevorzugt ist. Diese winklige Verbindung ist in den Figuren 8b, 9b und 10b dargestellt.

Wie in Figur 2 dargestellt, ist der Trichter 11 im Bereich der ersten Anlagefläche 12 dicker ausgebildet als in seiner angrenzenden Wandung. Dasselbe gilt für die zweite Anlagefläche 22 des Kragens 2. Diese Situation ist vergrössert in Figur 8a dargestellt.

Der Neigungswinkel β der Stufe, in welcher die zwei Wandungen in die zwei Anlageflächen 12, 22 übergehen, beträgt vorzugsweise 30° bis 70° und noch bevorzugter 45°. Die Erhöhung d der äusseren Oberfläche beträgt in diesem Verbindungsbereich 0.5 bis 1.5 mm. Die Wandstärken des Trichters 11 und des Kragens 2 sind mindestens im Verbindungsbereich gleich gross. IN den Beispielen der Figuren 10a und 10b sind sie es auch im angrenzenden Bereich.

Die Basiswandstärke des Trichters 11, d.h. die Wandstärke des mehrheitlichen Teils des Trichters 11, ist mit dem Bezugszeichen 15 versehen, die Basiswandstärke des Kragens 2 mit 25. Die verdickte erste Wandstärke des Trichters 11 weist das Bezugszeichen 14 auf, die verdickte zweite Wandstärke des Kragens 2 das Bezugszeichen 24. In Figur 8a sind die innere Oberfläche 13 des Trichters 11 und die innere Oberfläche 23 des Kragens 2 flach und miteinander fluchtend ausgebildet. Dies ist, wie oben beschrieben, in den hier dargestellten Ausführungsformen nicht der Fall.

In den Figuren 9a und 9b ist eine alternative Ausbildung des Verbindungsbereichs dargestellt. Hier verdickt sich lediglich der Trichter 11 stufenförmig und somit unstetig. Der Kragen 2 verdickt sich kontinuierlich.

In den Ausführungsformen gemäss den Figuren 10a und 10b ist keine Verdickung im Bereich der Verbindung vorhanden.

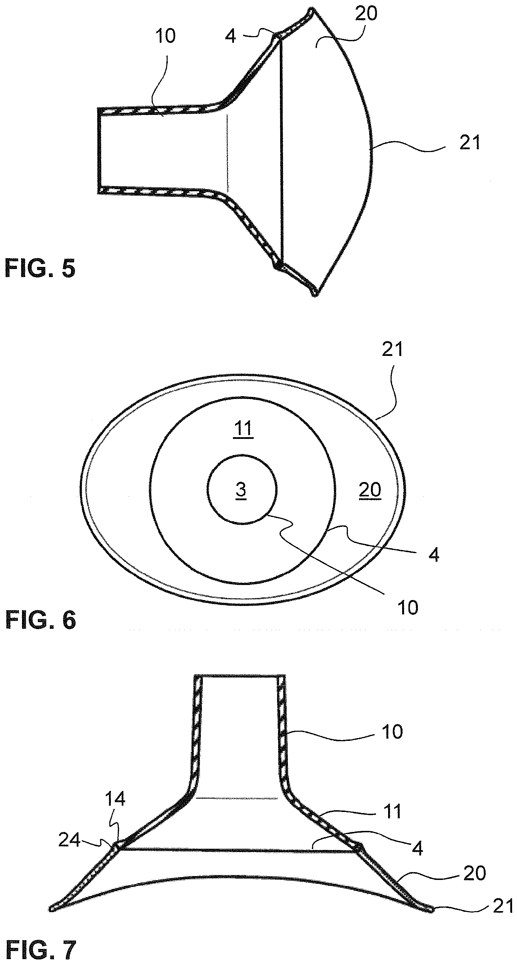

Im Ausführungsbeispiel gemäss Figur 4 werden der Trichter 11 und der Kragen 2 stetig dicker. Zudem ist der Kragen 2 kürzer ausgebildet als im ersten Ausführungsbeispiel. Im ersten Beispiel weist der Kragen 2 annähernd dieselbe Länge wie der Trichter 11 auf oder er weist zumindest mehr als die Hälfte der Länge des Trichters 11 auf. Im Beispiel gemäss Figur 4 beträgt seine Länge maximal die Hälfte der Länge des Trichters 11, gemessen jeweils entlang der äusseren Oberfläche. Dasselbe gilt für das Ausfuhrungsbeispiel gemäss den Figuren 5 bis 7. Im Ausführungsbeispiel gemäss den Figuren 5 bis 7 ist die Ellipse des Auflagerandes 21 flacher ausgebildet als diejenige im ersten Ausführungsbeispiel.

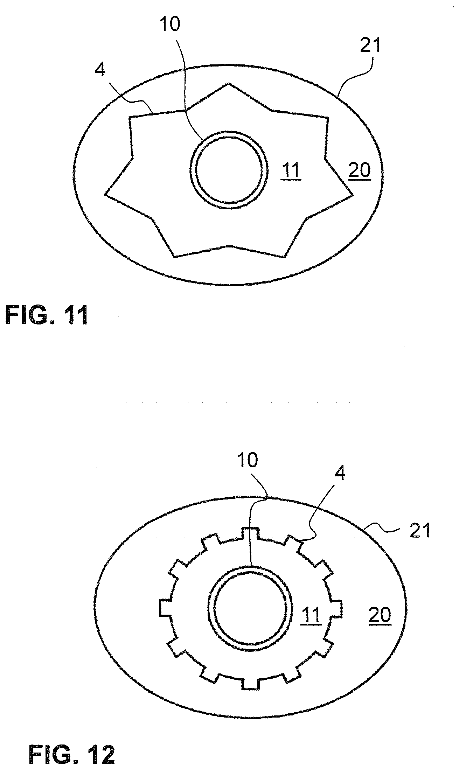

In den Figuren 11 und 12 sind zwei Varianten der Verbindungsnaht 4 dargestellt. In den oben beschriebenen Ausführungsformen ist die Verbindungsnaht 4 ein Kreis. In der Ausführungsform gemäss Figur 11 ist sie unstetig und weist eine Sternform auf. In Figur 12 ist sie ebenfalls unstetig. Sie ist hier mäanderförmig bzw. verzahnend. Diese Formen haben den Vorteil, dass der Verbund zwischen Kragen 2 und Trichter 11 verstärkt ist. Weitere Formen sind ebenfalls möglich.

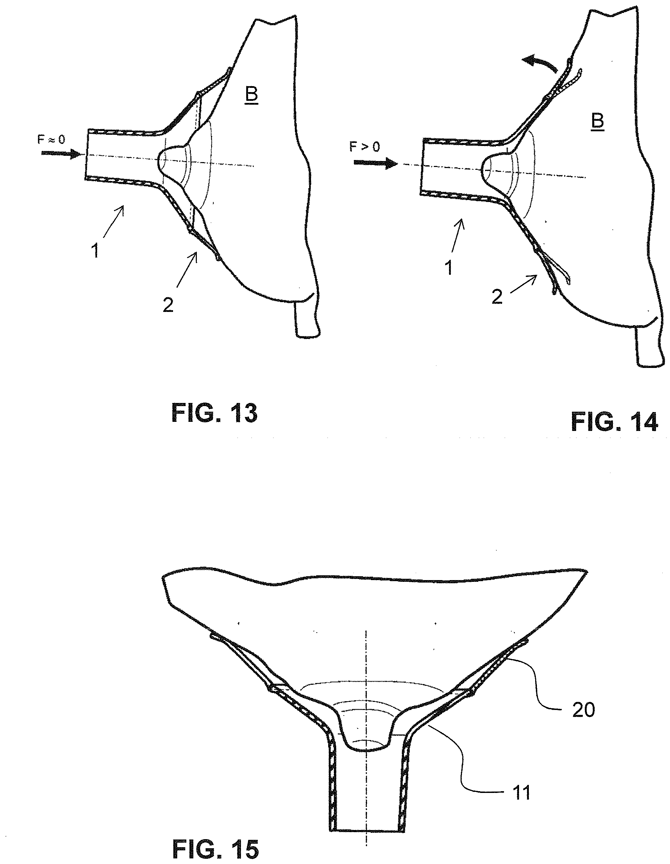

In den Figuren 13 bis 15 ist dargestellt, dass sich der Kragen 2 an die Brust B flexibel anpasst und sich sein Öffnungswinkel beim Anpressen an die Brust B vergrössert. In den Figuren 13 und 15 liegt noch kein Anpressdruck F vor und der Kragen 2 ist noch unbelastet. Der Kragen 2 ist noch nach innen geneigt. In Figur 14 wird die Brusthaube mit einer Kraft F grösser als Null gegen die Brust B gepresst. Der Kragen 2 biegt sich in Richtung des gebogenen Pfeils von der Brust B weg und der Öffnungswinkel vergrössert sich. Der Kragen 2 liegt mit seinem gesamten Umfang dichtend, aber ohne schmerzende Druckstellen und ohne übermässigen Druck an der Brust B an. Dank der nach innen gerichteten Neigung des Kragens im unbelasteten Zustand, genügt ein geringer Druck, damit die Brusthaube dichtend anliegt und sich der Anatomie der Brust anpasst.

Vorzugsweise ist die Länge des Kragens 2 so bemessen, dass er bei Gebrauch über seine gesamte Länge an der Brust anliegt. Dadurch wird vermieden, dass er sich bei angelegtem Unterdruck verformt. Die Brustwarze und die Areola der Brust liegen vorzugsweise im steifen Trichter, so dass beim Anlegen des Vakuums keine Verformung der Brusthaube in diesem Bereich stattfinden kann.

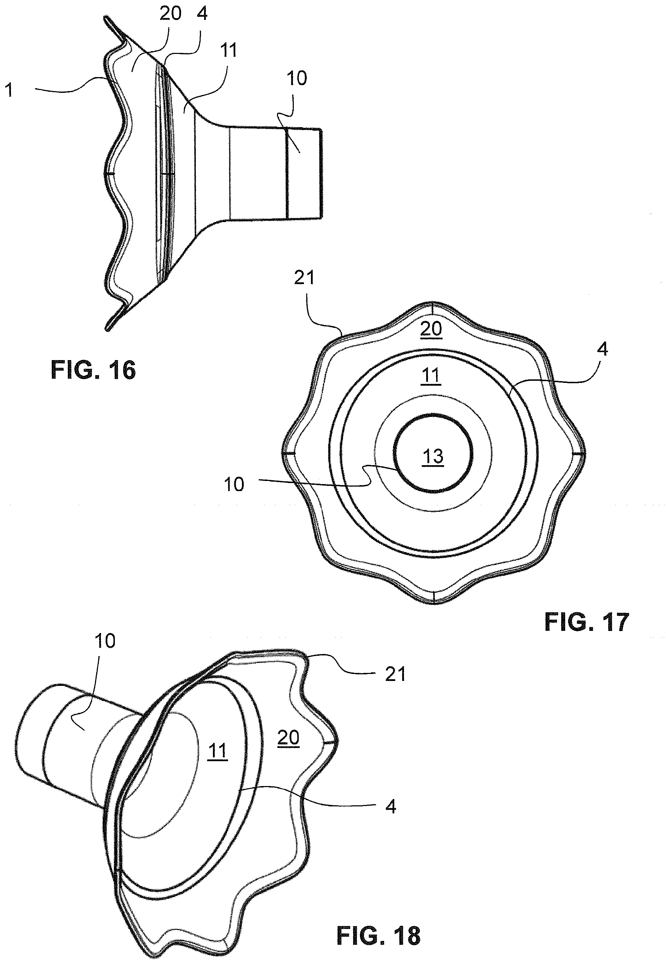

In den Figuren 16 bis 22 sind zwei weitere Ausführungsbeispiele dargestellt. Der Trichter 11 und der Grundkörper 20 des Kragens 2 sind wiederum ein gerader Kreiskegelstumpf. Der Kragen 20 weist jedoch einen Auflagerand 21 in Form einer Raumkurve auf, deren Projektion senkrecht zur Längsmittelachse L_{T} der Brusthaube einen in sich geschlossenen Polygonzug ergibt.

Der Auflagerand 21 in der Ausführungsform gemäss den Figuren 16 bis 18 ist ein 8-eckiger in sich geschlossener Polygonzug mit abgerundeten Ecken und nach innen gebogenen Seiten. Seine Projektion weist somit drei Symmetrieachsen auf und seine umhüllende Form ist ein Kreis. Der Auflagerand und der Kragen weisen drei entsprechende Symmetrieebenen auf.

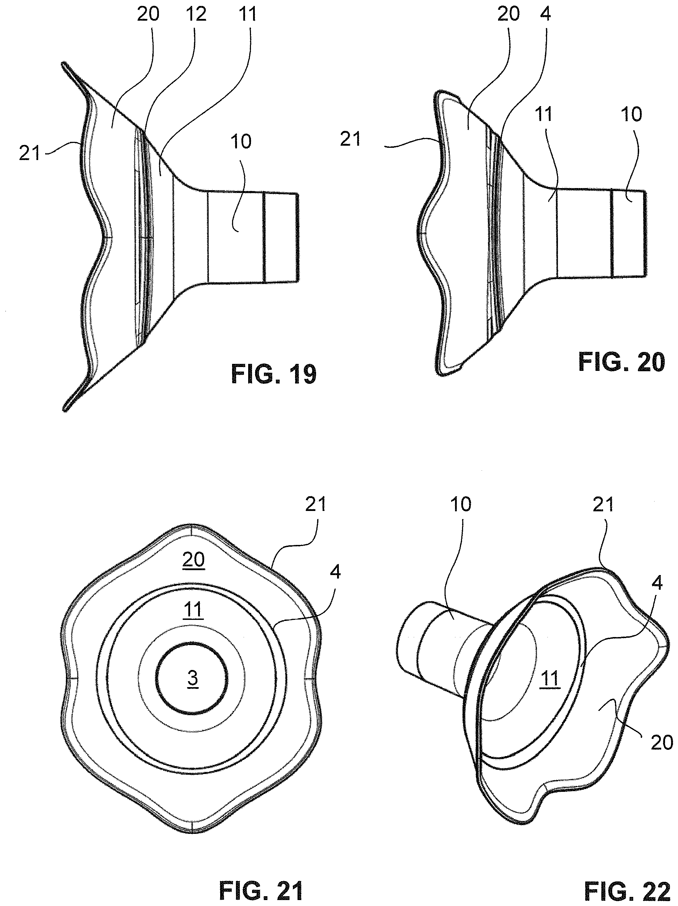

Der Auflagerand 21 in der Ausführungsform gemäss den Figuren 19 bis 22 ist ein 6-eckiger in sich geschlossener Polygonzug mit abgerundeten Ecken und nach innen gebogenen Seiten.

Die Auflageränder 21 gemäss diesen zwei Ausführungsbeispielen haben ein blumenförmiges Erscheinungsbild. Diese zwei Varianten können mit den bereits erwähnten verschiedenen Verbindungsnähten und Wandstärken, insbesondere nach den Figuren 8a bis 10b, versehen sein. Der Kragen 2 ist vorzugsweise auch jeweils nach innen geneigt oder er kann, wie in den Figuren 8a, 9a und 10a dargestellt, geradlinig angespritzt sein.

Die erfindungsgemässe Brusthaube lässt sich einfach und kostengünstig herstellen und ermöglicht trotzdem einen hohen Tragkomfort und eine dichte Auflage auf die Mutterbrust.

**BEZUGSZEICHENLISTE**

| | | | |
|---|---|---|---|
| 1 | Basisteil | | |
| 10 | Stutzen | 3 | Durchgang |
| 11 | Trichter | | |
| 12 | erste Anlagefläche | 4 | Verbindungsnaht |
| 13 | innere Oberfläche | | |
| 14 | erste Wandstärke | α₁ | erster Öffnungswinkel |
| 15 | erste Basiswandstärke | α₂ | zweiter Öffnungswinkel |
| | | β | Neigungswinkel |
| 2 | Kragen | d | Erhöhung |
| 20 | Grundkörper | L_{T} | Längsmittelachse |
| 21 | Auflagerand | S₁ | erste Symmetrieachse/ebene |
| 22 | zweite Anlagefläche | S₂ | zweite Symmetrieachse/ebene |
| 23 | innere Oberfläche | | |
| 24 | zweite Wandstärke | B | Mutterbrust |
| 25 | zweite Basiswandstärke | | |

## Patentansprüche

1. Brusthaube für eine Brustpumpe zum Abpumpen von menschlicher Muttermilch, wobei die Brusthaube ein steifes Basisteil (1) mit einem rohrförmigen Stutzen (10) und mit einem einstückig daran angeformten, sich vom Stutzen (10) weg erweiternden Trichter (11) aufweist zur Aufnahme einer Mutterbrust, wobei die Brusthaube ferner einen flexiblen Kragen (2) aufweist, welcher an einem dem Stutzen (10) abgewandten Ende des Trichters (11) am Trichter (11) angeordnet ist und welcher zur Anlage auf die Mutterbrust ausgebildet ist, wobei das genannte Ende des Trichters (11) eine erste Anlagefläche (12) bildet, wobei der Kragen (2) ein dem Trichter (11) zugewandtes Ende aufweist, welches eine zweite Anlagefläche (22) bildet,
wobei die erste und die zweite Anlagefläche (12, 22) in einer umlaufenden Verbindungsnaht (4) stumpf aneinander grenzen,
wobei der Trichter (11) im Bereich der Verbindungsnaht (4) eine erste Wandstärke (14) und der Kragen (2) im Bereich der Verbindungsnaht (4) eine zweite Wandstärke (24) aufweist und
wobei die erste Wandstärke (14) und die zweite Wandstärke (24) annähernd gleich gross sind,
**dadurch gekennzeichnet, dass** die erste Wandstärke (14) im Bereich der Verbindungsnaht (4) im Vergleich zu einer angrenzenden Wandstärke des Trichters (11) vergrössert ist und/oder dass die zweite Wandstärke (24) des Kragens (2) im Bereich der Verbindungsnaht (4) im Vergleich zu einer angrenzenden Wandstärke des Kragens (2) vergrössert ist.

2. Brusthaube nach Anspruch 1, wobei die erste Wandstärke (14) und die zweite Wandstärke (24) genau gleich gross sind.

3. Brusthaube nach einem der Ansprüche 1 oder 2, wobei der Trichter (11) einen ersten Öffnungswinkel (α₁) aufweist, wobei der Kragen (2) im unbelasteten Zustand einen zweiten Öffnungswinkel (α₂) aufweist und wobei der zweite Öffnungswinkel (α₂) kleiner ist als der erste Öffnungswinkel (α₁).

4. Brusthaube nach einem der Ansprüche 1 bis 3, wobei der Kragen (2) im unbelasteten Zustand in Bezug auf den Trichter (11) nach innen geneigt ist.

5. Brusthaube nach einem der Ansprüche 1 bis 4, wobei der Kragen (2) beim Anlegen an die Mutterbrust nach aussen drückbar und somit eine Aufnahmeöffnung des Kragens (2) vergrösserbar ist.

6. Brusthaube nach einem der Ansprüche 1 bis 5, wobei der Kragen (2) mindestens zwei oder genau zwei Symmetrieebenen (S₁, S₂) aufweist.

7. Brusthaube nach einem der Ansprüche 1 bis 6, wobei der Kragen (2) ein dem Trichter (11) abgewandtes Ende aufweist, welches einen Auflagerand (21) zur Auflage an die Mutterbrust bildet, wobei der Auflagerand (21) eine Raumkurve ist, welche nicht in einer Ebene verläuft.

8. Brusthaube nach Anspruch 7, wobei eine Projektion des Auflagenrands (21) in einer Ebene senkrecht zu einer Längsachse (L_{T}) der Brusthaube eine Ellipse ist.

9. Brusthaube nach Anspruch 7, wobei eine Projektion des Auflagenrands (21) in einer Ebene senkrecht zu einer Längsachse (L_{T}) der Brusthaube ein in sich geschlossener Polygonzug mit abgerundeten Ecken und nach innen geneigten Seiten ist.

10. Brusthaube nach einem der Ansprüche 1 bis 9, wobei der Kragen (2) eine gerader Kegelstumpf oder ein gerader Kreiskegelstumpf ist.

11. Brusthaube nach einem der Ansprüche 1 bis 10, wobei der Kragen (2) in seinem unbelasteten Zustand in Richtung der Längsmittelachse (L_{T}) der Brusthaube einen gleichbleibenden Öffnungswinkel (α₂ ) aufweist, mindestens bis in einen Bereich eines dem Trichter (11) abgewandten Endes des Kragens (2).

12. Brusthaube nach einem der Ansprüche 1 bis 11, wobei die Verbindungsnaht (4) in einer Ebene verläuft.

13. Brusthaube nach einem der Ansprüche 1 bis 12, wobei die Verbindungsnaht (4) eine unstetige Form aufweist.

14. Brusthaube nach einem der Ansprüche 1 bis 13, wobei der Trichter (11) ein Kreiskegelstumpf ist und der Kragen (2) ein dem Trichter (11) abgewandtes Ende aufweist, welches einen Auflagerand (21) zur Auflage auf die Mutterbrust bildet, wobei dieser Auflagerand (21) eine von einem Kreis abweichende Form aufweist.

## Claims

1. Breast shield for a breast pump for expressing human breast milk, wherein the breast shield comprises a rigid base part (1) with a tubular connection piece (10) and with a funnel (11), which is integrally moulded in one piece thereon and widens away from the connection piece (10), for receiving a mother's breast, wherein the breast shield additionally comprises a flexible collar (2) which is arranged on the funnel (11) at an end of the funnel (11) remote from the connection piece (10) and which is realized to be placed onto the mother's breast, wherein the named end of the funnel (11) forms a first contact surface (12), wherein the collar (2) comprises an end which faces the funnel (11) and forms a second contact surface (22),
wherein the first and the second contact surface (12, 22) adjoin one another in an obtuse manner in a circumferential connecting joint (4),
wherein the funnel (11) comprises a first wall thickness (14) in the region of the connecting joint (4) and the collar (2) comprises a second wall thickness (24) in the region of the connecting joint (4) and
wherein the first wall thickness (14) and the second wall thickness (24) are approximately the same size,
**characterized in that** the first wall thickness (14) is increased in the region of the connecting joint (4) in comparison to an adjoining wall thickness of the funnel (11) and/or in that the second wall thickness (24) of the collar (2) is increased in the region of the connecting joint (4) in comparison to an adjoining wall thickness of the collar (2).

2. Breast shield according to Claim 1, wherein the first wall thickness (14) and the second wall thickness (24) are precisely the same size.

3. Breast shield according to one of Claims 1 or 2, wherein the funnel (11) comprises a first opening angle (α₁), wherein the collar (2) comprises a second opening angle (α₂) in the non-loaded state and wherein the second opening angle (α₂) is smaller than the first opening angle (α₁).

4. Breast shield according to one of Claims 1 to 3, wherein the collar (2) is inclined inwards with reference to the funnel (11) in the non-loaded state.

5. Breast shield according to one of Claims 1 to 4, wherein the collar (2) is pressable outwardly when placed onto the mother's breast and consequently a receiving opening of the collar (2) is enlargeable.

6. Breast shield according to one of Claims 1 to 5, wherein the collar (2) comprises at least two or precisely two planes of symmetry (S₁, S₂).

7. Breast shield according to one of Claims 1 to 6, wherein the collar (2) comprises an end remote from the funnel (11) which forms a lay-on edge (21) to be placed onto the mother's breast, wherein the lay-on edge (21) is a three-dimensional curve which does not extend in one plane.

8. Breast shield according to Claim 7, wherein a projection of the lay-on edge (21) in a plane perpendicular to a longitudinal axis (L_{T}) of the breast shield is an ellipse.

9. Breast shield according to Claim 7, wherein a projection of the lay-on edge (21) in a plane perpendicular to a longitudinal axis (L_{T}) of the breast shield is a traverse which is closed per se and has rounded corners and sides which incline inwards.

10. Breast shield according to one of Claims 1 to 9, wherein the collar (2) is a straight truncated cone or a straight circular truncated one.

11. Breast shield according to one of Claims 1 to 10, wherein the collar (2) in its non-loaded state comprises a constant opening angle (α₂) in the direction of the longitudinal center axis (L_{T}) of the breast shield, at least as far as into a region of an end of the collar (2) remote from the funnel (11).

12. Breast shield according to one of Claims 1 to 11, wherein the connecting joint (4) extends in one plane.

13. Breast shield according to one of Claims 1 to 12, wherein the connecting joint (4) comprises an inconstant form.

14. Breast shield according to one of Claims 1 to 13, wherein the funnel (11) is a circular truncated cone and the collar (2) comprises an end which is remote from the funnel (11) and forms a lay-on edge (21) to be placed onto the mother's breast, wherein said lay-on edge (21) comprises a form which deviates from a circle.

## Revendications

1. Téterelle pour un tire-lait pour l'extraction de lait maternel humain, la téterelle présentant une partie de base rigide (1) comprenant un raccord tubulaire (10) et comprenant un entonnoir (11) formé d'un seul tenant sur celui-ci, s'élargissant à partir du raccord (10), pour la réception d'un sein maternel, la téterelle présentant en outre une collerette flexible (2), qui est agencée sur l'entonnoir (11) à une extrémité de l'entonnoir (11) détournée du raccord (10) et qui est configurée pour s'appuyer sur le sein maternel, ladite extrémité de l'entonnoir (11) formant une première surface d'application (12), la collerette (2) présentant une extrémité tournée vers l'entonnoir (11), qui forme une deuxième surface d'application (22),
la première et la deuxième surface d'application (12, 22) étant contiguës l'une à l'autre bout à bout dans un joint d'assemblage périphérique (4),
l'entonnoir (11) présentant une première épaisseur de paroi (14) dans la zone du joint d'assemblage (4) et la collerette(2) présentant une deuxième épaisseur de paroi (24) dans la zone du joint d'assemblage (4) et
la première épaisseur de paroi (14) et la deuxième épaisseur de paroi (24) étant approximativement égales,
**caractérisée en ce que**
la première épaisseur de paroi (14) dans la zone du joint d'assemblage (4) est agrandie en comparaison d'une épaisseur de paroi adjacente de l'entonnoir (11) et/ou **en ce que** la deuxième épaisseur de paroi (24) de la collerette (2) dans la zone du joint d'assemblage (4) est agrandie en comparaison d'une épaisseur de paroi adjacente de la collerette(2).

2. Téterelle selon la revendication 1, dans laquelle la première épaisseur de paroi (14) et la deuxième épaisseur de paroi (24) sont exactement égales.

3. Téterelle selon l'une quelconque des revendications 1 ou 2, dans laquelle l'entonnoir (11) présente un premier angle d'ouverture (α₁), dans laquelle la collerette(2) présente un deuxième angle d'ouverture (α₂) à l'état non sollicité et dans laquelle le deuxième angle d'ouverture (α₂) est inférieur au premier angle d'ouverture (α₁).

4. Téterelle selon l'une quelconque des revendications 1 à 3, dans laquelle la collerette (2) est inclinée vers l'intérieur par rapport à l'entonnoir (11) à l'état non sollicité.

5. Téterelle selon l'une quelconque des revendications 1 à 4, dans laquelle la collerette(2) peut être pressée vers l'extérieur lors de la mise en place sur le sein maternel et une ouverture de réception de la collerette(2) peut ainsi être agrandie.

6. Téterelle selon l'une quelconque des revendications 1 à 5, dans laquelle la collerette(2) présente au moins deux ou exactement deux plans de symétrie (S₁, S₂).

7. Téterelle selon l'une quelconque des revendications 1 à 6, dans laquelle la collerette (2) présente une extrémité détournée de l'entonnoir (11), qui forme un bord d'appui (21) pour l'appui sur le sein maternel, le bord d'appui (21) étant une courbe spatiale qui ne s'étend pas dans un plan.

8. Téterelle selon la revendication 7, dans laquelle une projection du bord d'appui (21) dans un plan perpendiculaire à un axe longitudinal (L_{T}) de la téterelle est une ellipse.

9. Téterelle selon la revendication 7, dans laquelle une projection du bord d'appui (21) dans un plan perpendiculaire à un axe longitudinal (L_{T}) de la téterelle est un tracé polygonal fermé sur lui-même ayant des coins arrondis et des côtés inclinés vers l'intérieur.

10. Téterelle selon l'une quelconque des revendications 1 à 9, dans laquelle la collerette (2) est un cône tronqué droit ou un cône tronqué circulaire droit.

11. Téterelle selon l'une quelconque des revendications 1 à 10, dans laquelle la collerette (2) présente, à son état non sollicité, un angle d'ouverture constant (α₂) en direction de l'axe médian longitudinal (L_{T}) de la téterelle, au moins jusque dans une zone d'une extrémité de la collerette (2) détournée de l'entonnoir (11).

12. Téterelle selon l'une quelconque des revendications 1 à 11, dans laquelle le joint d'assemblage (4) s'étend dans un plan.

13. Téterelle selon l'une quelconque des revendications 1 à 12, dans laquelle le joint d'assemblage (4) présente une forme discontinue.

14. Téterelle selon l'une quelconque des revendications 1 à 13, dans laquelle l'entonnoir (11) est un cône tronqué circulaire et la collerette (2) présente une extrémité détournée de l'entonnoir (11), qui forme un bord d'appui (21) pour l'appui sur le sein maternel, ce bord d'appui (21) présentant une forme différente d'un cercle.
